# EUROPEAN PATENT SPECIFICATION

(11) **EP 2 907 808 B1**
(45) Date of publication and mention of the grant of the patent: **02.01.2019**
(21) Application number: 13845206.5
(22) Date of filing: 09.09.2013
(51) Int. Cl.: C07D 251/60

(54) **METHOD FOR COOLING A MELAMINE MELT**
VERFAHREN ZUR KÜHLUNG EINER MELAMINSCHMELZE
PROCÉDÉ DE REFROIDISSEMENT D'UN BAIN DE FUSION DE MÉLAMINE

(30) Priority: 11.10.2012 RU 2012143644
(43) Date of publication of application: 19.08.2015
(73) Proprietor: Otkrytoe Aktsionernoe Obschestvo Research & Design Institute Of Urea And Organic Synthesis Products (OAO NIIK), Nizhny Novgorod Region 606008 (RU)
(72) Inventor: NIKOLAEV, Evgeny Yuryevich, Nizhny Novgorog Region g. Dzerzhinsk 606037 (RU); SKUDIN, Alexei Georgievich, Nizhny Novgorog Region g. Dzerzhinsk 606009 (RU); PECHNIKOVA, Galina Nikolaevna, Nizhny Novgorog Region Volodarskv District Dubki Village Krasnaya Gorka Settlement 606070 (RU); PROKOPYEV, Aleksandr Alekseevich, Nizhny Novgorog Region g. Volodarsk 606070 (RU); KUZNETSOV, Nikolai Mikhailovich, Nizhny Novgorog Region g. Dzerzhinsk 606000 (RU); KOSTIN, Oleg Nikolaevich, g. Nizhny Novgorod 603010 (RU); ESIN, Igor Veniaminovich, Nizhny Novgorog Region g. Dzerzhinsk 606000 (RU)
(74) Representative: Vossius & Partner Patentanwälte Rechtsanwälte mbB
(86) International application number: PCT/RU2013/000784
(87) International publication number: WO 2014/058347

(56) References cited:
- EP-A2- 0 747 366
- WO-A1-99/38852
- EA-B1- 003 233
- RU-A- 2006 134 260
- RU-C2- 2 161 608
- US-A- 3 132 143
- US-A- 3 308 123
- US-A- 4 565 867
- US-A- 4 565 867

## Description

### Field of the Invention

The invention relates to processes of melamine production by pyrolysis of urea in a high-pressure non-catalytic process, namely, to methods for cooling melamine melt using water and aqueous solutions.

### Background of the Invention

In the process of producing melamine from urea, a large amount of gaseous by-products, ammonia and carbon dioxide, is released. They constitute approximately 70% of the feedstock urea weight. Melamine and off-gases having a temperature of 350°C to 400°C are to be cooled before further processing to lower temperatures, typically up to 200°C and below.

Last time, separation of products of melamine synthesis reactor before cooling or quenching thereof to liquid unpurified melamine melt and gas phase is preferred, with further separate treatment. This method allows more effective use of heat energy of reaction mass simplifying its further treatment. There are several ways to cool melamine melt using water and aqueous solutions.

The known is a method for cooling melamine melt with an aqueous ammonia solution with a concentration of more than 10% at a temperature of 160-170°C to obtain an aqueous solution of melamine (EA 005993, C07D 251/60, 2005). In this case, melamine melt is fed to the bottom part of the cooling column, where it is vigorously stirred at a temperature of 160-170°C with water and ammonia solution, coming to the same part of the column.

After cooling, melamine is purified from condensation products by exposing aqueous-ammonia melamine solution formed for a period less than 30 minutes, and then separating the melamine by crystallization.

This cooling method provides an efficient cooling of the melamine melt, but the use of ammonia in the cooling process makes necessary to perform further aqueous treatment in an ammonia solution. As a consequence, the stages of melamine crystallization and centrifugation become more complicated, and the use of special devices is required to prevent emission of ammonia to the environment. Moreover, it requires energy-intensive removal of ammonia from the mother liquor and further ammonia condensation.

The known is a method for cooling melamine melt using aqueous alkali solution with a concentration of 0.05-0.5% at a temperature of 100-150°C to obtain alkali-containing aqueous melamine solution (EP 1444214, C07D 251/62, 2004). Sodium hydroxide or potassium hydroxide is used as alkali. The melamine melt is cooled in a vessel of a special design. To obtain uniform distribution and ensure rapid cooling of melamine melt, aqueous alkali solution and melamine melt is fed simultaneously to the upper part of the vessel via separate spraying devices or doublet injector.

An aqueous alkali-containing melamine solution formed by cooling the melt is kept at the bottom part of the cooling vessel within 5-60 minutes to decompose the products of condensation. Melamine is then separated by crystallization.

The cooling of melamine melt having a temperature above 350°C with the use of aqueous alkali solution causes significant hydrolysis of melamine. It results in large melamine loss and formation of ammeline and ammelide as hydrolysis products. Ammeline and ammelide formation requires increased consumption of alkali to convert ammeline and ammelide into water soluble salts.

The known is a method of cooling melamine melt with water having a purity of above 95% at a temperature of 170-220°C to obtain aqueous melamine solution (RU 2367656, C07D 251/60, C07D 251/62, 2009).

After melamine melt cooling, ammonia and carbon dioxide are removed from the melamine solution formed. The process of gas removal is performed in a distillation column by feeding steam to the bottom part of the column at practically same temperature as cooling temperature, or higher temperature. To decompose the products of condensation, alkali is added to melamine solution which is substantially free of ammonia and carbon dioxide, and the solution is kept within 5-60 minutes, preferably 20-40 minutes. Melamine is then separated by crystallization.

The cooling of melamine melt by water in the absence of alkali reduces melamine hydrolysis. However, long residence time of melamine solution in a distillation column under high temperature causes substantial loss of melamine due to hydrolysis, additional consumption of alkali being required to keep hydrolysis products dissolved as described above.

From the technical point of view, the method that is the closest to the proposed one is a known method comprising melamine melt cooling by water to a temperature of 25-300°C, preferably, to 50-200°C, in the cooling zone with transition of melamine to the solid state and formation of aqueous melamine suspension, and removal of melamine from the cooling zone in the form of aqueous suspension (EA 003233, C07D 251/62, C07D 251/60, 2003). Melamine melt and cooling water are sprayed at the upper part of the cooling vessel; the resulting melamine suspension is preferably stirred and continuously removed from the bottom part of the vessel.

It is specified in EA 003233 specification that melamine with sufficiently high degree of purity can be obtained by simple filtration or centrifuging of the suspension and further drying. However, as described in examples of the specification, the purity of melamine does not exceed value of 99.1%. This degree of purity is not sufficient for most applications where higher degree of purity is required, at least 99.8%. To achieve higher purity, additional recrystallization is supposed. It is also possible to add alkali and keep the solution before crystallization to remove the undesirable melamine by-products.

The amount of water to form melamine suspension when cooling the melt is not specified in the examples of implementing the known method. However, this value is very important. If the concentration of melamine in the suspension is less than 10%, aqueous phase contains considerable amount of melamine. Thus, losses of melamine with mother liquor are great. Moreover, melamine precipitates from the mother liquor on the walls of vessels and piping as the liquor is cooled. Higher concentration of the melamine suspension can cause transportation problems due to high viscosity of the suspension and its tendency to stick. Thus, handling of melamine suspensions complicates the process.

Less water is required to form melamine suspension if compared with cooling process forming aqueous melamine solutions. However, it is not possible to avoid losses of melamine and formation of hydrolysis products in the cooling process, because part of melamine passes to aqueous phase, and dissolved melamine is hydrolyzed, especially much at a temperature above 180°C.

The mentioned disadvantages make difficult to implement in industry the method of melamine melt cooling with water to obtain melamine suspension.

### Summary of the Invention

The technical problem which should be solved by invention is an improvement of the method of melamine melt cooling with water which enables reduction of melamine losses.

The technical result which can be obtained using the invention is lowering degree of melamine hydrolysis at the stage of melamine melt cooling as well as simplification of the process.

To achieve the technical result, the method is proposed in the claims for cooling melamine melt with water to temperature of 130-230°C in a cooling zone with formation of solid melamine and removing melamine from the cooling zone characterized in that cooling is performed with water evaporation and formation of solid melamine-water vapor gaseous suspension, and melamine is removed from the cooling zone in a form of solid melamine-water vapor gaseous suspension.

To improve the process of removal from the cooling zone, nitrogen, air or a mixture thereof can be added to the formed solid melamine-water vapor gaseous suspension.

In the prior art, no solutions having features identical to the features of the proposed invention are specified.

### Brief Description of Drawings

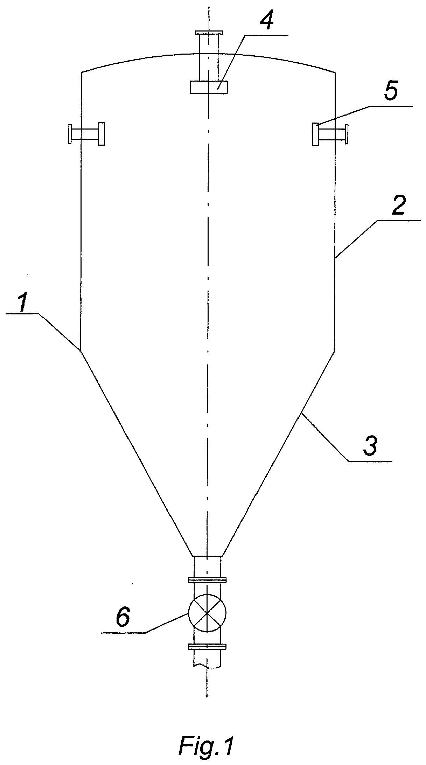

Cooling vessel for the proposed method of cooling melamine melt is presented schematically in Fig. 1.

### Description of the Preferred Embodiments

In accordance with Fig. 1, the cooling vessel comprises a body 1 consisting of an upper cylindrical part 2 and a cone-shaped bottom 3. Along the central axis of the upper part 2 of the body 1, nozzle 4 is being provided to enable the input of melamine melt. In the walls of upper cylindrical part 2 of the body 1, eight nozzles 5 for cooling water input are located symmetrically and perpendicularly to unit axis. In the cone-shaped bottom 3, there is a throttle valve 6 for removal of solid melamine-water vapour gaseous suspension from the vessel.

The bottom 3 of the body is cone-shaped to prevent the formation of dead zones and facilitate the removal of solid melamine-water vapour gaseous suspension from the vessel. The bottom may have means for nitrogen and/or air inlet, not shown in Fig. 1, enabling their input to the solid melamine-water vapour gaseous suspension prior to its removal from the cooling zone.

The invention is illustrated by the following examples of the implementing proposed method with the use of the cooling vessel shown in Fig. 1.

### Example 1.

The melamine melt is cooled in a cooling vessel shown in Fig. 1. Melamine melt with a temperature of 380°C in an amount of 20 kg/h is sprayed into cylindrical part 2 of the body 1 of the cooling unit through the nozzle 4. Simultaneously, cooling water in an amount of 3.3 kg/h is sprayed into the cylindrical part 2 through the side nozzles 5. Water evaporates, and solid melamine-water vapour gaseous suspension with a temperature of 230°C is formed. It passes through the cone-shaped bottom 3 and is removed from the body 1 through the throttle valve 6 for further purification and melamine separation.

The chemical analysis carried out by high performance liquid chromatography (HPLC) showed that the content of hydrolysis products in the melamine melt after cooling has not changed.

### Example 2.

The cooling of melamine melt is carried out according to Example 1 with the only difference that the amount of cooling water sprayed into cylindrical part 2 through the side nozzles 5 is 4.6 kg/h. Water evaporates to form solid melamine-water vapour gaseous suspension with a temperature of 130°C. After passing the suspension through the cone-shaped bottom 3, it is removed from the body 1 through the throttle valve 6 for further purification and melamine separation.

The chemical analysis carried out by HPLC showed that hydrolysis products content in melamine after melt cooling is not changed.

Inspection revealed no sticking of melamine on the walls of the vessel, at least for four months of continuous operation.

The proposed method of cooling melamine melt with water has a number of significant advantages in comparison with the prior art.
1. The process of evaporation of cooling water when contacting with melamine melt excludes the presence of a liquid aqueous phase in the cooling zone. Solidified melamine does not contact with liquid water and form aqueous solution. It makes hydrolysis of the melamine at the melamine melt cooling stage practically impossible, so there are no losses of melamine and no formation of hydrolysis products requiring additional consumption of alkali during melamine purification.
2. It is much more convenient to remove and transport the formed solid melamine-water vapour gaseous suspension from the cooling vessel, than remove aqueous melamine suspension, especially concentrated suspension, which greatly simplifies the process.
3. The amount of water used to cool melamine melt is reduced.

### Industrial Applicability

The invention may be used in the industrial production of melamine.

## Claims

1. A method of cooling melamine melt obtainable by pyrolysis of urea in a high-pressure non-catalytic process by water to a temperature of 130 to 230°C in the cooling zone with the transition of melamine to a solid state and removal of melamine from the cooling zone **characterized in that** the cooling is carried out with evaporation of water and formation of solid melamine-water vapour gaseous suspension, and melamine is removed from the cooling zone in the form of solid melamine-water vapour gaseous suspension,
wherein the cooling is conducted in a cooling vessel comprising a body 1 consisting of an upper cylindrical part 2 and a cone-shaped bottom 3, a nozzle 4 being provided along the central axis of the upper cylindrical part 2 of the body 1, eight nozzles 5 for cooling water input being located symmetrically and perpendicularly to unit axis in the walls of upper cylindrical part 2 of the body 1 and a throttle valve 6 being provided in the cone-shaped bottom 3, and
wherein the melamine melt is input via nozzle 4, water is input via the nozzles 5 for cooling water input and the solid melamine-water vapour gaseous suspension is removed from the vessel via throttle valve 6.

2. The method according to claim 1 **characterized in that** nitrogen and/or air is introduced to the solid melamine-water vapour gaseous suspension via means for nitrogen and/or air inlet provided in the cone-shaped bottom 3 before removal of the solid melamine-water vapour gaseous suspension from the cooling zone.

## Patentansprüche

1. Ein Verfahren zum Abkühlen von Melaminschmelze, erhältlich durch Pyrolyse von Harnstoff in einem nichtkatalytischen Hochdruckverfahren durch Wasser auf eine Temperatur von 130 bis 230°C in der Kühlzone, wobei Melamin in einen festen Zustand versetzt wird und Melamin aus der Kühlzone entfernt wird, **dadurch gekennzeichnet, dass** das Abkühlen durch Verdunsten von Wasser und Bildung einer gasförmigen Suspension von festem Melamin und Wasserdampf erfolgt und Melamin in Form von gasförmiger Suspension von festem Melamin und Wasserdampf aus der Kühlzone entfernt wird,
wobei das Abkühlen in einem Kühlgefäß ausgeführt wird, das einen Körper 1, bestehend aus einem oberen zylindrischen Teil 2 und einem kegelförmigen Boden 3, einer Düse 4, die entlang der Mittelachse des oberen zylindrischen Teils 2 des Körpers 1 bereitgestellt ist, acht Düsen 5 zum Einlass von Kühlwasser, die symmetrisch und senkrecht zur Einheitsachse in den Wänden des oberen zylindrischen Teils 2 des Körpers 1 angeordnet sind und einer Drosselklappe 6, die im kegelförmigen Boden 3 bereitgestellt ist, umfasst, und
wobei die Melaminschmelze über Düse 4 eingelassen wird, Wasser über die Düsen 5 zum Einlass von Kühlwasser eingelassen wird und die gasförmige Suspension von festem Melamin und Wasserdampf aus dem Gefäß über Drosselklappe 6 entfernt wird.

2. Das Verfahren nach Anspruch 1, **dadurch gekennzeichnet, dass** Stickstoff und/oder Luft in die gasförmige Suspension von festem Melamin und Wasserdampf über Mittel zum Einlass von Stickstoff und/oder Luft, die im kegelförmigen Boden 3 bereitgestellt sind, vor dem Entfernen der gasförmige Suspension von festem Melamin und Wasserdampf aus der Kühlzone eingeführt wird.

## Revendications

1. Méthode de refroidissement d'une masse fondue de mélamine pouvant être obtenue par pyrolyse d'urée dans un procédé non catalytique haute pression au moyen d'eau à une température de 130 à 230 °C dans la zone de refroidissement avec la transition de la mélamine à un état solide et le retrait de la mélamine de la zone de refroidissement **caractérisée en ce que** le refroidissement est effectué par évaporation d'eau et formation d'une suspension gazeuse de mélamine solide et de vapeur d'eau, et la mélamine est retirée de la zone de refroidissement sous la forme d'une suspension gazeuse de mélamine solide et de vapeur d'eau,
dans laquelle le refroidissement est effectué dans une cuve de refroidissement comprenant un corps 1 consistant en une partie cylindrique supérieure 2 et un fond en forme de cône 3, une buse 4 étant disposée le long de l'axe central de la partie cylindrique supérieure 2 du corps 1, huit buses 5 pour l'entrée d'eau de refroidissement étant situées symétriquement et perpendiculairement à un axe unitaire dans les parois de la partie cylindrique supérieure 2 du corps 1, et une soupape d'étranglement 6 étant disposée dans le fond en forme de cône 3, et
dans laquelle la masse fondue de mélamine est introduite via la buse 4, l'eau est introduite via les buses 5 pour l'entrée d'eau de refroidissement, et la suspension gazeuse de mélamine solide et de vapeur d'eau est retirée de la cuve via la soupape d'étranglement 6.

2. Méthode selon la revendication 1, **caractérisée en ce que** de l'azote et/ou de l'air sont introduits dans la suspension gazeuse de mélamine solide et de vapeur d'eau via des moyens d'entrée d'azote et/ou d'air disposés dans le fond en forme de cône 3 avant le retrait de la suspension gazeuse de mélamine solide et de vapeur d'eau de la zone de refroidissement.
